(19) Europäisches Patentamt
European Patent Office
Office européen des brevets

(11) **EP 4 287 118 A1**

(12) **EUROPEAN PATENT APPLICATION**
published in accordance with Art. 153(4) EPC

(43) Date of publication:
06.12.2023 Bulletin 2023/49

(51) International Patent Classification (IPC):
*G06T 7/30* (2017.01)    *G06T 7/593* (2017.01)
*A61B 6/00* (2006.01)    *A61B 6/14* (2006.01)
*A61B 6/03* (2006.01)    *A61B 5/00* (2006.01)
*A61C 9/00* (2006.01)

(21) Application number: 22742780.4

(22) Date of filing: 17.01.2022

(86) International application number:
PCT/KR2022/000826

(87) International publication number:
WO 2022/158804 (28.07.2022 Gazette 2022/30)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(30) Priority: 20.01.2021 KR 20210007926

(71) Applicant: HDX Will Corp.
Seoul 03162 (KR)

(72) Inventors:
• JUNG, Hong
Seoul 03162 (KR)
• LEE, Sung Min
Bucheon-si, Gyeonggi-do 14553 (KR)
• SONG, Bo Hyun
Seoul 06723 (KR)

(74) Representative: BOVARD AG
Patent- und Markenanwälte
Optingenstrasse 16
3013 Bern (CH)

(54) **DEVICE AND METHOD FOR MATCHING THREE-DIMENSIONAL INTRA-ORAL SCAN DATA USING COMPUTED TOMOGRAPHY IMAGE**

(57) Disclosed is a device for matching three-dimensional intra-oral scan data, the device comprising a computed tomography (CT) image depth map generation unit, a depth map coordinate difference information determination unit, a depth map correction unit, and an intra-oral scan model recovery unit. The CT image depth map generation unit generates a depth map of a CT frame from a CT image by using information about a single scan frame of the scan data. The depth map coordinate difference information determination unit determines depth map coordinate difference information between the depth map of the CT frame and a depth map of the scan frame. The depth map correction unit corrects the depth map of the scan frame on the basis of the depth map coordinate difference information. The intra-oral scan model recovery unit recovers a three-dimensional intra-oral scan model on the basis of the corrected depth map of the scan frame.

FIG. 2

**100**

EP 4 287 118 A1

**Description**

[Technical Field]

The present invention relates to a device and method for matching three-dimensional (3D) intraoral scan data.

[Background Art]

As the digital dentistry market expands, in the dental field including orthodontics and implants, three-dimensional (3D) computed tomography (CT) images and 3D intraoral scan data are highly utilized for accurate diagnosis planning and treatment. In particular, scan data has an advantage of higher tooth crown precision than a CT image, and a CT image has an advantage of providing dental root information, and thus the scan data and the CT image are often used together. However, since a CT image is an image reconstructed by photographing the entire tooth, geometric distortion does not occur. On the other hand, since intraoral scan data is an image reconstructed by matching multiple pieces of data acquired by locally photographing the entire oral cavity, geometric distortion occurs due to a matching error. This causes a geometric difference between the CT image and the intraoral scan data.

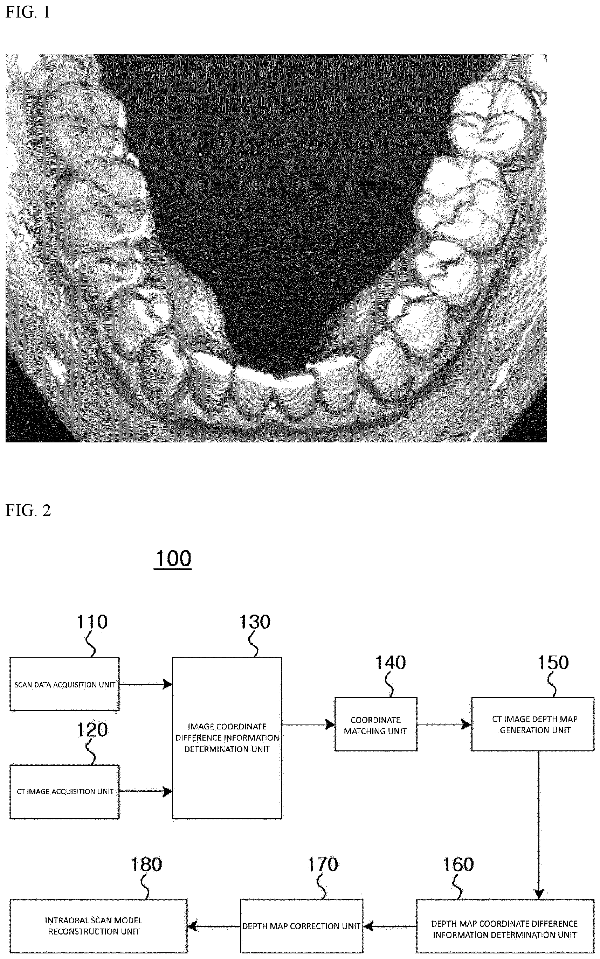

FIG. 1 shows a difference between scan data and a CT image.

As shown in FIG. 1, there is a geometric difference between the scan data and the CT image.

3D intraoral scan data is generated by matching point clouds included in each of a plurality of frames that are generated by locally scanning each part of the oral cavity with a scanner into one in a 3D coordinate system. In order to match 3D point clouds, it is necessary to calculate relative positions of the point clouds generated for each frame by obtaining a relative rotation amount and movement amount of a scan camera. However, when a small error occurs in a process of obtaining a position and orientation of the camera, even when an error between frames of a frame pair is small, errors for several frames are accumulated, resulting in geometric distortion in a reconstructed oral cavity model.

[Related Art Document]

[Patent Document]

(Patent Document 1) Korean Patent Registration No. 10-1911327

[Disclosure]

[Technical Problem]

The present invention is directed to providing a method and device for matching three-dimensional (3D) intraoral scan data that can reduce geometric distortion of a scan model.

[Technical Solution]

One aspect of the present invention provides a three-dimensional (3D) method of matching intraoral scan data, which includes generating a depth map of a computed tomography (CT) frame from a CT image using information about one scan frame of scan data; determining depth map coordinate difference information between the depth map of the CT frame and a depth map of the scan frame; correcting the depth map of the scan frame on the basis of the depth map coordinate difference information; and reconstructing a 3D intraoral scan model on the basis of the corrected depth map of the scan frame.

The depth map coordinate difference information may include a depth map rotation matrix representing a degree of rotation between the depth map of the CT frame and the depth map of the scan frame, and a depth map translation vector representing a degree of translation between the depth map of the CT frame and the depth map of the scan frame.

The depth map rotation matrix and the depth map translation vector may minimize a sum of differences between the depth map of the CT frame and the depth map of the scan frame.

The method of matching the 3D intraoral scan data may further include determining image coordinate difference information between the scan data and the CT image; and matching a coordinate system of the scan data to a coordinate system of the CT image on the basis of the image coordinate difference information.

The image coordinate difference information may include an image rotation matrix representing a degree of rotation between the scan data and the CT image, and an image translation vector representing a degree of translation between the scan data and the CT image.

The image rotation matrix and the image translation vector may minimize a sum of differences between the scan data and the CT image.

The method of matching the 3D intraoral scan data may further include acquiring scan data for an oral cavity of a patient using an intraoral scanner; and acquiring a CT image of the oral cavity of the patient using CT equipment.

The 3D intraoral scan model may be reconstructed by merging point clouds of corrected depth maps of a plurality of frames of the scan data in three dimensions and averaging common parts.

The information about the scan frame may include information about a position and direction of a camera of the scan frame.

A size of the depth map of the CT frame may be identical to a size of the depth map of the scan frame.

Another aspect of the present invention provides a computer program stored in a computer-readable medium, wherein, when a command of the computer program is executed, the method is performed.

Still another aspect of the present invention provides a device for matching 3D intraoral scan data, which includes a CT image depth map generation unit configured to generate a depth map of a CT frame from a CT image using information about one scan frame of scan data; a depth map coordinate difference information determination unit configured to determine depth map coordinate difference information between the depth map of the CT frame and a depth map of the scan frame; a depth map correction unit configured to correct the depth map of the scan frame on the basis of the depth map coordinate difference information; and an intraoral scan model reconstruction unit configured to reconstruct a 3D intraoral scan model on the basis of the corrected depth map of the scan frame.

The depth map coordinate difference information may include a depth map rotation matrix representing a degree of rotation between the depth map of the CT frame and the depth map of the scan frame, and a depth map translation vector representing a degree of translation between the depth map of the CT frame and the depth map of the scan frame.

The depth map rotation matrix and the depth map translation vector may minimize a sum of differences between the depth map of the CT frame and the depth map of the scan frame.

Yet another aspect of the present invention provides an intraoral scanner which includes the device for matching 3D intraoral scan data according to the embodiment of the present invention.

Yet another aspect of the present invention provides a CT device which includes the device for matching 3D intraoral scan data according to the embodiment of the present invention.

[Advantageous Effects]

According to the embodiment of the present invention, by locally matching frames obtained from a scanner to a computed tomography (CT) image using a CT image that does not have geometric distortion to reconstruct a scan model, it is possible to reduce the geometric distortion of the scan model.

[Description of Drawings]

FIG. 1 shows a difference between scan data and a computed tomography (CT) image.

FIG. 2 is a block diagram of a device for matching three-dimensional (3D) intraoral scan data according to an embodiment of the present invention.

FIG. 3 is a flowchart illustrating operations of a device for matching 3D intraoral scan data according to an embodiment of the present invention.

FIG. 4 is a diagram showing an operation process of a device for matching 3D intraoral scan data according to an embodiment of the present invention through an oral cavity image.

FIG. 5 shows a real frame depth map and a CT image depth map according to an embodiment of the present invention.

[Modes of the Invention]

Hereinafter, embodiments of the present invention that can be easily performed by those skilled in the art will be described in detail with reference to the accompanying drawings. In addition, parts irrelevant to description are omitted in the drawings in order to clearly explain the present invention.

Next, a device for matching three-dimensional (3D) intraoral scan data according to an embodiment of the present invention will be described with reference to FIG. 2.

FIG. 2 is a block diagram of a device for matching 3D intraoral scan data according to an embodiment of the present invention.

As shown in FIG. 2, a device 100 for matching 3D intraoral scan data includes a scan data acquisition unit 110, a computed tomography (CT) image acquisition unit 120, an image coordinate difference information determination unit 130, a coordinate matching unit 140, a CT image depth map generation unit 150, a depth map coordinate difference information determination unit 160, a depth map correction unit 170, and an intraoral scan model reconstruction unit 180.

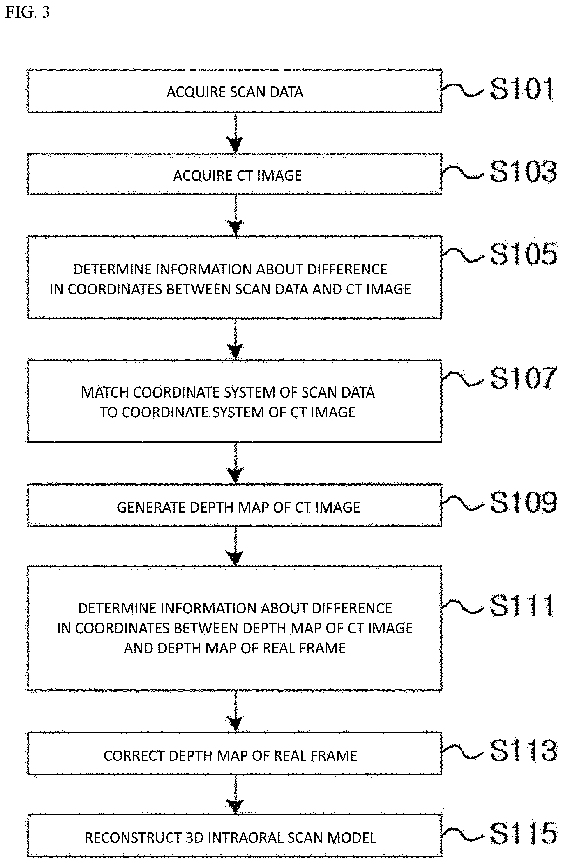

The operation of each component of the device 100 for matching 3D intraoral scan data will be described with reference to FIGS. 3 to 5.

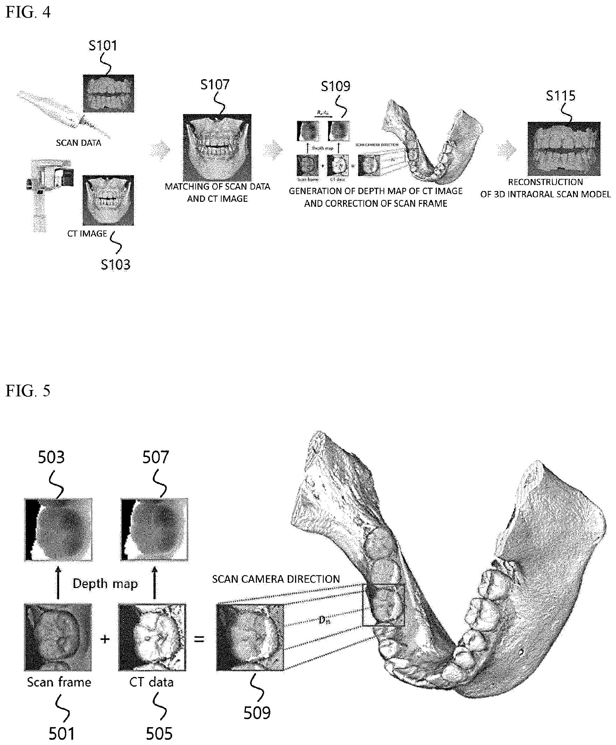

FIG. 3 is a flowchart illustrating operations of the device for matching 3D intraoral scan data according to the embodiment of the present invention, and FIG. 4 is a diagram showing an operation process of the device for matching 3D intraoral scan data according to the embodiment of the present invention through an oral cavity image.

The scan data acquisition unit 110 acquires scan data for an oral cavity of a patient using an intraoral scanner (S101). The scan data may include a plurality of scan frames. In this specification, since the scan frame is acquired using a scanner, the scan frame is also referred to as a real frame.

The CT image acquisition unit 120 acquires a CT image of the oral cavity of the patient using CT equipment 20 (S103).

The image coordinate difference information determination unit 130 determines information about a difference in coordinates between the scan data and the CT image (S105). The information about the difference in coordinates between the scan data and the CT image may be determined using an iterative closest point (ICP) algorithm as shown in Equation 1, and may include a rotation matrix R and a translation vector t that minimize an error E(R,t). The error E(R,t) denotes the sum of differences between point values determined by the rotation matrix R and the translation vector t, wherein the differences between the point values are differences between point values of the scan data and point values of the CT image.

[Equation 1]

$$E(R,t) = \sum_{i=1}^{N_s} \sum_{j=1}^{N_c} w_{i,j} \| s_i - (Rc_j + t) \|^2$$

In Equation 1, Ns denotes the total number of points of the scan data, and Nc denotes the total number of points of the CT image. i denotes a position of one point of the scan data, $s_i$ denotes a value of a point at a position i of the scan data, j denotes a position of one point of the CT image, and $c_j$ denotes a value of a point at a position j of the CT image. $\omega_{i,j}$ denotes a weight, has a value of 1 when a distance between $s_i$ and $c_j$ is closest, and has a value of 0 otherwise.

The coordinate matching unit 140 matches a coordinate system of the scan data to a coordinate system of the CT image by matching the scan data and the CT image on the basis of the information about the difference in coordinates between the scan data and the CT image (S107).

The CT image depth map generation unit 150 generates a depth map of a plurality of CT frames from the CT image using a camera position and direction of each of the plurality of frames obtained in the process of reconstructing the scan data (S109). In this case, a size of the depth map of the CT image may be identical to a size of a real frame depth map of the scan data.

FIG. 5 shows a real frame depth map and a CT image depth map according to an embodiment of the present invention.

As shown in FIG. 5, a scan frame depth map 503 may be generated from a scan frame 501. Further, a CT frame depth map 507 may be generated from a CT frame 505 corresponding to the scan frame 501 on the basis of a camera position and direction of the scan frame 501. Meanwhile, when the scan frame 501 and the CT frame 505 are matched, a frame 509 corresponding to a scan camera direction $D_n$ may be generated.

Again, FIG. 3 will be described.

A difference in position and direction between the CT frame depth map and the real frame depth map means that there is an error in the camera position and direction of each scan frame obtained in the process of reconstructing the scan data. Therefore, in the embodiment of the present invention, the difference in position and orientation between the CT image depth map and the real frame depth map is corrected using an ICP algorithm.

The depth map coordinate difference information determination unit 160 determines information about a difference in coordinates between a depth map of a CT image and a depth map of a real frame (S111). The information about the difference in coordinates between the depth map of the CT image and the depth map of the real frame may be determined using an ICP algorithm as shown in Equation 2, and may include a rotation matrix $R_n$ and a translation vector $t_n$ for an $n^{th}$ real frame that minimize an error $E_n$. The error $E_n$ denotes the sum of differences between point values determined by the rotation matrix $R_n$ and the translation vector $t_n$, wherein the differences between the point values are differences between point values of the depth map of the real frame and point values of the depth map of the CT frame.

[Equation 2]

$$E_n(R_n, t_n) = \sum_{i=1}^{N_{D_n^C}} \sum_{j=1}^{N_{D_n^S}} w_{i,j} \| d_{n,i}^C - (R_n d_{n,j}^S + t_n) \|^2$$

Here, $D_n^S$ denotes a depth map of an $n^{th}$ real frame, $D_n^C$ denotes a depth map of an $n^{th}$ CT image, $N_{D_n^S}$ denotes the total number of pixels of the depth map of the $n^{th}$ real frame, and $N_{D_n^C}$ denotes the total number of pixels of the depth map of the $n^{th}$ CT frame. $d_{n,i}^C$ denotes a depth value of one point of the depth map of the CT image, and $d_{n,i}^S$ denotes a depth value of one point of the depth map of the scan data. $\omega_{i,j}$ denotes a weight, has a value of 1 when a distance between $d_{n,i}^S$ and $d_{n,i}^C$ is closest, and has a value of 0 otherwise.

The depth map correction unit 170 corrects the depth map of the real frame on the basis of the information about the difference in coordinates between the depth maps (S113). As shown in Equation 3, the real frame depth map correction unit 160 may correct the depth map $D_n^S$ of the $n^{th}$ real frame on the basis of the rotation matrix $R_n$ and the translation vector $t_n$ to generate a corrected depth map $D_n^{s'}$.

[Equation 3]

$$D_n^{s'} = R_n(D_n^s) + t_n$$

In this way, when the position and direction of the existing scan camera are corrected using the rotation matrix and translation vector that are obtained for each frame and the depth map of the real frame is corrected, the position and direction of each real frame become the same as those of the CT image, and thus errors in the camera position and direction of each scan frame may be corrected.

The intraoral scan model reconstruction unit 180 reconstructs a 3D intraoral scan model by merging point clouds of the corrected depth map of the real frame in three dimensions and averaging common parts (S 1 15).

The device for matching 3D intraoral scan data according to the embodiment of the present invention may be included as a module in a CT device or an intraoral scanner, or may be implemented in software in which CT data and/or intraoral scanner data are used.

While the present invention has been described with reference to the embodiment illustrated in the accompanying drawings, the embodiment should be considered in a descriptive sense only, and it should be understood by those skilled in the art that various alterations and equivalent other embodiments may be made. Therefore, the scope of the present invention should be defined by only the following claims.

**Claims**

1. A method of matching three-dimensional (3D) intraoral scan data, comprising:

generating a depth map of a computed tomography (CT) frame from a CT image using information about one scan frame of scan data;
determining depth map coordinate difference information between the depth map of the CT frame and a depth map of the scan frame;
correcting the depth map of the scan frame on the basis of the depth map coordinate difference information; and
reconstructing a 3D intraoral scan model on the basis of the corrected depth map of the scan frame.

2. The method of claim 1, wherein the depth map coordinate difference information includes a depth map rotation matrix representing a degree of rotation between the depth map of the CT frame and the depth map of the scan frame, and a depth map translation vector representing a degree of translation between the depth map of the CT frame and the depth map of the scan frame.

3. The method of claim 2, wherein the depth map rotation matrix and the depth map translation vector minimize a sum of differences between the depth map of the CT frame and the depth map of the scan frame.

4. The method of claim 1, further comprising:

   determining image coordinate difference information between the scan data and the CT image; and
   matching a coordinate system of the scan data to a coordinate system of the CT image on the basis of the image coordinate difference information.

5. The method of claim 4, wherein the image coordinate difference information includes an image rotation matrix representing a degree of rotation between the scan data and the CT image, and an image translation vector representing a degree of translation between the scan data and the CT image.

6. The method of claim 5, wherein the image rotation matrix and the image translation vector minimize a sum of differences between the scan data and the CT image.

7. The method of claim 1, further comprising:

   acquiring scan data for an oral cavity of a patient using an intraoral scanner; and
   acquiring a CT image of the oral cavity of the patient using CT equipment.

8. The method of claim 1, wherein the 3D intraoral scan model is reconstructed by merging point clouds of corrected depth maps of a plurality of frames of the scan data in three dimensions and averaging common parts.

9. The method of claim 1, wherein the information about the scan frame includes information about a position and direction of a camera of the scan frame.

10. The method of claim 1, wherein a size of the depth map of the CT frame is identical to a size of the depth map of the scan frame.

11. A device for matching three-dimensional (3D) intraoral scan data, comprising:

   a computed tomography (CT) image depth map generation unit configured to generate a depth map of a CT frame from a CT image using information about one scan frame of scan data;
   a depth map coordinate difference information determination unit configured to determine depth map coordinate difference information between the depth map of the CT frame and a depth map of the scan frame;
   a depth map correction unit configured to correct the depth map of the scan frame on the basis of the depth map coordinate difference information; and
   an intraoral scan model reconstruction unit configured to reconstruct a 3D intraoral scan model on the basis of the corrected depth map of the scan frame.

12. The device of claim 11, wherein the depth map coordinate difference information includes a depth map rotation matrix representing a degree of rotation between the depth map of the CT frame and the depth map of the scan frame, and a depth map translation vector representing a degree of translation between the depth map of the CT frame and the depth map of the scan frame.

13. The device of claim 12, wherein the depth map rotation matrix and the depth map translation vector minimize a sum of differences between the depth map of the CT frame and the depth map of the scan frame.

14. An intraoral scanner comprising the device for matching three-dimensional (3D) intraoral scan data according to any one claim of claims 11 to 13.

15. A computed tomography (CT) device comprising the device for matching three-dimensional (3D) intraoral scan data according to any one claim of claims 11 to 13.

16. A computer program stored in a computer-readable medium, wherein, when a command of the computer program is executed, the method according to any one claim of claims 1 to 10 is performed.

FIG. 1

FIG. 2

**100**

FIG. 3

ACQUIRE SCAN DATA $\sim$ S101

ACQUIRE CT IMAGE $\sim$ S103

DETERMINE INFORMATION ABOUT DIFFERENCE IN COORDINATES BETWEEN SCAN DATA AND CT IMAGE $\sim$ S105

MATCH COORDINATE SYSTEM OF SCAN DATA TO COORDINATE SYSTEM OF CT IMAGE $\sim$ S107

GENERATE DEPTH MAP OF CT IMAGE $\sim$ S109

DETERMINE INFORMATION ABOUT DIFFERENCE IN COORDINATES BETWEEN DEPTH MAP OF CT IMAGE AND DEPTH MAP OF REAL FRAME $\sim$ S111

CORRECT DEPTH MAP OF REAL FRAME $\sim$ S113

RECONSTRUCT 3D INTRAORAL SCAN MODEL $\sim$ S115

FIG. 4

S101

SCAN DATA

CT IMAGE

S103

S107

MATCHING OF SCAN DATA
AND CT IMAGE

S109

GENERATION OF DEPTH MAP OF CT IMAGE
AND CORRECTION OF SCAN FRAME

S115

RECONSTRUCTION
OF 3D INTRAORAL SCAN MODEL

FIG. 5

503

507

Depth map

Scan frame

501

+

CT data

505

=

SCAN CAMERA DIRECTION

$D_n$

509

## INTERNATIONAL SEARCH REPORT

| International application No. |
| --- |
| **PCT/KR2022/000826** |

### A. CLASSIFICATION OF SUBJECT MATTER

**G06T 7/30**(2017.01)i; **G06T 7/593**(2017.01)i; **A61B 6/00**(2006.01)i; **A61B 6/14**(2006.01)i; **A61B 6/03**(2006.01)i; **A61B 5/00**(2006.01)i; **A61C 9/00**(2006.01)i

According to International Patent Classification (IPC) or to both national classification and IPC

### B. FIELDS SEARCHED

Minimum documentation searched (classification system followed by classification symbols)

G06T 7/30(2017.01); A61B 1/24(2006.01); A61B 5/00(2006.01); A61C 19/04(2006.01); A61C 9/00(2006.01); G06T 11/00(2006.01); G06T 3/60(2006.01); G06T 5/00(2006.01); G06T 7/593(2017.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 컴퓨터 단층촬영(Computed Tomography, CT), 3차원(3-dimensional), 구강(oral), 스캔 데이터(scan data), 정합(registration)

### C. DOCUMENTS CONSIDERED TO BE RELEVANT

| Category* | Citation of document, with indication, where appropriate, of the relevant passages | Relevant to claim No. |
| --- | --- | --- |
| X Y A | ZHOU, Xinwen et al. A Method for Tooth Model Reconstruction Based on Integration of Multimodal Images. Hindawi Journal of Healthcare Engineering. Volume 2018, Article ID 4950131, 20 June 2018. See pages 1-4; and figure 1. | 1-3,7,9-16 8 4-6 |
| Y | KR 10-2056910 B1 (DOF INC.) 17 December 2019 (2019-12-17) See paragraph [0046]. | 8 |
| A | KR 10-2016-0004864 A (INS BIO CO., LTD.) 13 January 2016 (2016-01-13) See paragraphs [0028]-[0031]; and figure 8. | 1-16 |
| A | KR 10-2019-0101694 A (SEOUL NATIONAL UNIVERSITY R&DB FOUNDATION) 02 September 2019 (2019-09-02) See paragraphs [0050]-[0052]. | 1-16 |

☑ Further documents are listed in the continuation of Box C.    ☑ See patent family annex.

| * | Special categories of cited documents: |
| --- | --- |
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
| --- | --- |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| **29 April 2022** | **02 May 2022** |

| Name and mailing address of the ISA/KR | Authorized officer |
| --- | --- |
| **Korean Intellectual Property Office** **Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

**INTERNATIONAL SEARCH REPORT**

| International application No. |
| --- |
| **PCT/KR2022/000826** |

**C.     DOCUMENTS CONSIDERED TO BE RELEVANT**

| Category* | Citation of document, with indication, where appropriate, of the relevant passages | Relevant to claim No. |
| --- | --- | --- |
| A | KR 10-2018-0088061 A (EWOO SOFT CO., LTD. et al.) 03 August 2018 (2018-08-03)<br>See claim 1. | 1-16 |
| PX | KR 10-2273437 B1 (HDX WILL CORP.) 07 July 2021 (2021-07-07)<br>※This document is a published earlier application that serves as a basis for claiming priority of the present international application. | 1-16 |

Form PCT/ISA/210 (second sheet) (July 2019)

| Patent document cited in search report | | | Publication date (day/month/year) | Patent family member(s) | | | Publication date (day/month/year) |
|---|---|---|---|---|---|---|---|
| KR | 10-2056910 | B1 | 17 December 2019 | CN | 113329713 | A | 31 August 2021 |
| | | | | EP | 3900666 | A1 | 27 October 2021 |
| | | | | WO | 2020-130598 | A1 | 25 June 2020 |
| KR | 10-2016-0004864 | A | 13 January 2016 | WO | 2016-003257 | A2 | 07 January 2016 |
| | | | | WO | 2016-003257 | A3 | 25 February 2016 |
| KR | 10-2019-0101694 | A | 02 September 2019 | WO | 2019-164093 | A1 | 29 August 2019 |
| KR | 10-2018-0088061 | A | 03 August 2018 | | None | | |
| KR | 10-2273437 | B1 | 07 July 2021 | | None | | |

Form PCT/ISA/210 (patent family annex) (July 2019)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 101911327